# EUROPEAN PATENT APPLICATION

(11) **EP 3 150 696 A1**
(43) Date of publication of application: **05.04.2017**
(21) Application number: 16190763.9
(22) Date of filing: 27.09.2016
(51) Int. Cl.: C12M 3/00, C12M 1/22, C12M 1/12

(54) **APPARATUS FOR PRODUCING CELL MASS SHEET AND METHOD FOR PRODUCING CELL MASS SHEET**

(30) Priority: 30.09.2015 JP 2015194799; 30.09.2015 JP 2015194800
(71) Applicant: Shibuya Corporation, Kanazawa-shi, Ishikawa 920-8681 (JP)
(72) Inventor: YONEDA, Kenji, Kanazawa-shi, Ishikawa 920-8681 (JP); KOSHIDA, Ichiro, Kanazawa-shi, Ishikawa 920-8681 (JP)
(74) Representative: Hodsdon, Stephen James

(57) **Abstract**

An apparatus 1 for producing a cell mass sheet is structured so as to produce a cell mass sheet 4 in which a plurality of cell masses 2 that have been planarly arranged on a mounting surface 13a in a culture container 3 are fused to each other by being cultured.

The apparatus includes guide means G that has a plurality of accommodating portions Ga which can accommodate the cell masses 2 therein, and suction nozzles 8 (supply means) that supply the cell masses 2 into the accommodating portions Ga of the guide means G, which are arranged on the mounting surface 13a; and is structured so that the suction nozzles 8 array the cell masses 2 on the mounting surface 13a, and after that, the guide means G retracts from the above the mounting surface 13a.

The apparatus can produce a cell mass sheet that has a constant shape and also a uniform thickness.

## Description

### BACKGROUND OF THE INVENTION

### Field of the Invention

The present invention relates to an apparatus for producing a cell mass sheet and a method for producing the cell mass sheet, and specifically relates to the apparatus for producing the cell mass sheet in which a plurality of cell masses that have been planarly arranged on a mounting surface in a culture container fuse to each other by being cultured, and the method for producing the cell mass sheet.

### Description of the Related Art

In regenerative medical techniques of renaturing vital functions that have received damage, by using a stem cell or the like, a cell sheet is used that is formed of cells which are cultured into high density. The cell sheet is extremely thin, and accordingly a method for producing a layered sheet of cultured cells is known in which several cell sheets are layered (Japanese Patent No. 4921353).

In addition, on the other hand, a method for producing sheet-like cell masses (cell mass sheet) having a thickness of 50 to 300 am is known, in which cell masses (spheroids) that have been formed each into an approximately spherical shape by being cultured are arrayed in a state of coming in contact with each other are fused to each other by being subjected to float culture (Japanese Patent No. 5523830).

Furthermore, a method for producing a three-dimensional structure of cells is known, which makes a support formed of a thread-like body or a needle-like body penetrate cell masses (Japanese Patent No. 4517125), as a method of arranging cell masses in an arbitrary space.

However, in the case of layering the cell sheets as in Japanese Patent No. 4921353, the cell sheet is difficult to handle and the automation has been difficult, because of being extremely thin.

In addition, in the case of intending to obtain the cell mass sheet by subjecting the cell masses to float culture as in Japanese Patent No. 5523830, the cell masses are irregularly fused to each other, and accordingly there have been problems that the shape of the obtained cell mass sheet is not constant, and also the thickness becomes nonuniform.

Furthermore, in the case of making the support formed of the thread-like body or the needle-like body penetrate the cell masses as in Japanese Patent No. 4517125, there has been a possibility of giving damage to the cell.

With respect to such problems, the present invention provides an apparatus for producing a cell mass sheet, which can produce a cell mass sheet having a constant shape and also a uniform thickness, and provides a method for producing the cell mass sheet.

### SUMMARY OF THE INVENTION

Specifically, an apparatus for producing a cell mass sheet according to the invention of claim 1 is the apparatus for producing a cell mass sheet, which produces a cell mass sheet in which a plurality of cell masses that have been planarly arranged on a mounting surface in a culture container are fused to each other by being cultured, and is characterized in that:
guide means that has a plurality of accommodating portions which can individually accommodate the cell masses therein, and supply means that supplies the cell masses into the accommodating portions of the guide means which has been placed on the mounting surface;
wherein the supply means arrays the cell masses on the mounting surface, and then the guide means is retracted from above the mounting surface.

A method for producing a cell mass sheet according to the invention of claim 10 is the method for producing a cell mass sheet, which produces a cell mass sheet in which a plurality of cell masses that have been planarly arranged on a mounting surface in a culture container are fused to each other by being cultured, and is characterized by including:
a guide placing step of mounting guide means that has accommodating portions regularly formed therein which can individually accommodate the cell masses therein, on the mounting surface;
a guide retracting step of making the guide means retract in a state in which the cell masses are arrayed on the mounting surface; and
a culture step of culturing the cell masses that are arrayed on the mounting surface, and making the cell masses fused to each other.

According to the invention according to claim 1, the apparatus makes the supply means accommodate the cell masses in the accommodating portions of the guide means that is placed on the mounting surface, and thereby can regularly array the cell masses according to the accommodating portion.

In this state, the guide means retracts from above the mounting surface, and thereby the apparatus keeps the state in which the cell masses are regularly arrayed, accordingly can produce a cell mass sheet having the same shape as that of the time when the cell masses have been arrayed by the guide means by being cultured in that state, and can make the thickness thereof constant.

According to the invention according to claim 10, the guide means accommodates the cell masses in the accommodating portions that are formed therein, thereby can regularly array the cell masses on the mounting surface of the culture container, and does not give damage to the cells at the time.

Because the cell masses are cultured in a state of being thus regularly arrayed, the cell masses are fused to each other in a state of being arrayed, and the cell mass sheet can be produced into a shape in which the cell masses are arrayed by the guide means.

### BRIEF DESCRIPTION OF THE DRAWINGS

FIG. 1 is block diagrams of an apparatus for producing a cell mass sheet according to a first embodiment;
FIG. 2 is a cross-sectional view of an accommodating container and a suction nozzle;
FIG. 3 shows perspective views of a culture tray and a culture container;
FIG. 4 is a plan view of the culture tray and the culture container;
FIG. 5 is side views of a culture container supporting section;
FIG. 6 is a side view of inspection means;
FIG. 7 shows side views of culture liquid supply means and cell mass sheet moving means;
FIG. 8 is a plan view of a culture tray which is usable in the first embodiment;
FIG. 9 is a plan view of the culture tray which is usable in the first embodiment, and a side view of a pin;
FIG. 10 is a plan view of the culture tray which is usable in the first embodiment;
FIG. 11 shows side views of the culture tray and the culture container which are usable in the first embodiment;
FIG. 12 is a side view of the culture liquid supply means which is usable in the first embodiment;
FIG. 13 shows block diagrams of an apparatus for producing a cell mass sheet according to a second embodiment;
FIG. 14 is plan views of a culture container;
FIG. 15 is a side view of the culture container;
FIG. 16 is a plan view for describing a holder;
FIG. 17 shows side views of cell mass sheet moving means;
FIG. 18 is a plan view of a culture container which is usable in the second embodiment;
FIG. 19 is a front view of an isolator and an incubator in which the apparatus for producing the cell mass sheet is provided; and
FIG. 20 is a block diagram of an apparatus for producing a cell mass sheet according to a third embodiment.

### DETAILED DESCRIPTION OF THE PREFERRED EMBODIMENTS

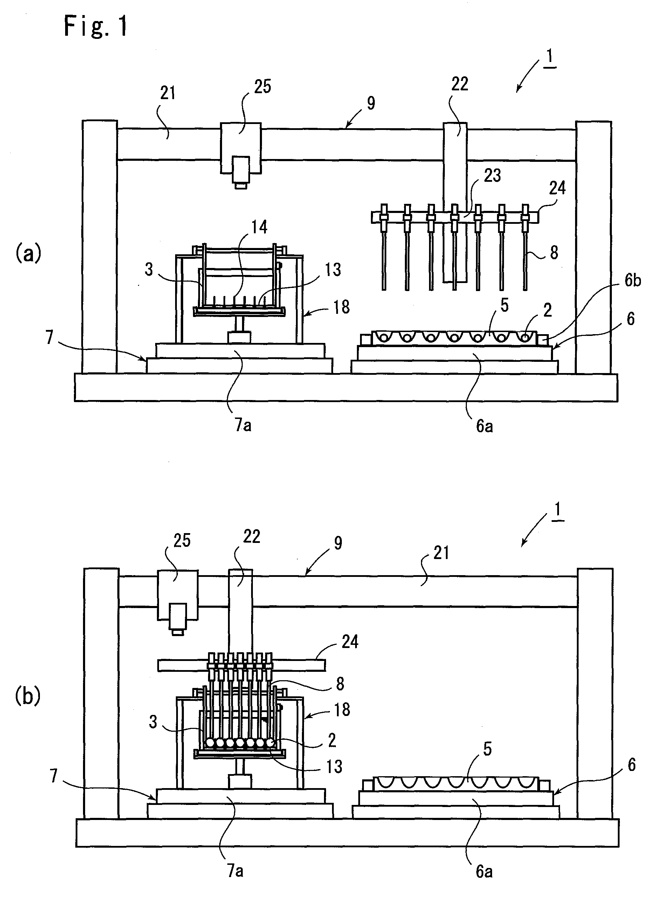

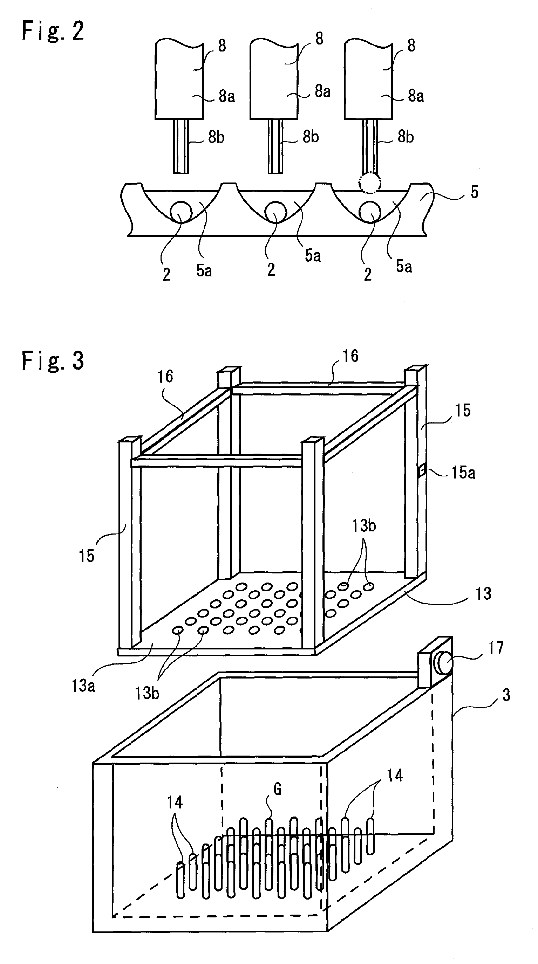

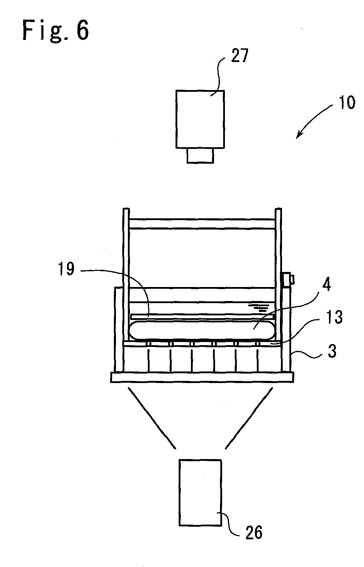

Illustrated embodiments will be described below. FIG. 1 to FIG. 7 are views for describing an apparatus 1 for producing a cell mass sheet according to a first embodiment, which is structured so as to produce a cell mass sheet 4 (see FIG. 6) in which a plurality of cell masses 2 (spheroids: see FIG. 2) are planarly arranged on a flat mounting surface that is provided in a culture container 3, and the above-described cell masses 2 are fused to each other by being cultured.

The above-described cell masses 2 can be produced, for instance, by a method which is disclosed in Japanese Patent No. 4517125. Specifically, when the cells are seeded and cultured in a container of which the inner surface is non-adhesive, the cells seek scaffolds, adhere to each other, and thereby form small cell masses. The small cell masses are further fused and thereby the cell masses 2 are formed that have an approximately spherical shape of which the outer diameter has a dimension of approximately less than 500 am.

More efficiently, the cell masses 2 are cultured in a non-adhesive well (accommodating portion having approximately hemispherical shape) in a well plate, and thereby can be more easily obtained. Incidentally, the method for producing the cell mass 2 is not limited to the above method, and can be produced by various known methods such as a rotary culture method of charging a cell suspension liquid into a rotating culture liquid, a method of charging a cell suspension liquid in a test tube and making the cells settle by a centrifugal separator, and a method of culturing cells with the use of alginate beads.

Thus, the cell mass 2 means a cell aggregate that is formed of cells which have aggregated and agglomerated to each other, and has an approximately spherical shape of which the outer diameter has a dimension of approximately 100 to 500 µm. The cell mass sheet 4 in the present invention is a sheet in which such a plurality of cell masses are fused to form a sheet shape. As for the thickness, the sheet can be produced which has the thickness of approximately 100 to 500 µm depending on the dimension of the cell mass 2 to be used, and the planer size can be adjusted by the number of the cell masses 2 to be arrayed.

The above-described cell mass sheet 4 that is produced in this way is different from a cell sheet that is obtained by culturing single cells into a sheet shape by planarly culturing a cell suspension liquid.

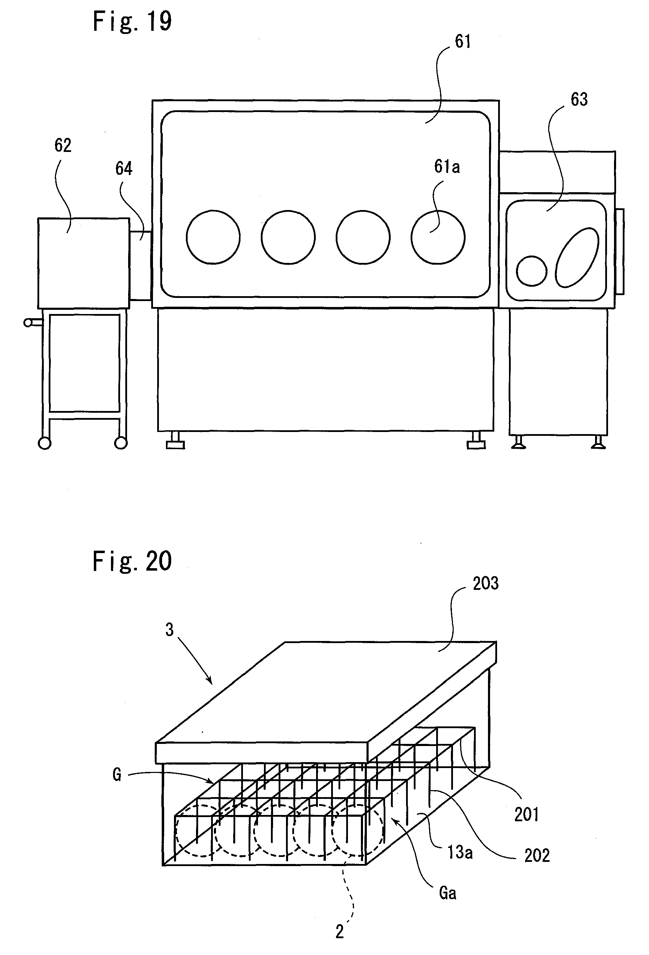

The apparatus 1 for producing a cell mass sheet in the present embodiment is provided in the inner parts of an isolator 61 of which the inner part is kept in a sterile condition, and of an incubator 62 which is provided so as to be capable of being connected to the isolator 61, which are shown in FIG. 19. In addition, a pass box 63 for decontaminating an object to be carried in is provided in the isolator 61.

FIG. 1 shows a structure provided in the inner part of the above-described isolator 61. The structure includes: an accommodating container supporting section 6 that supports the accommodating container 5 which accommodates the cell mass 2 therein; a culture container supporting section 7 that supports the culture container 3 in which the cell mass sheet 4 is cultured; suction nozzles 8 that act as a plurality of holding means each of which holds the above-described cell mass 2; nozzle moving means 9 that acts as moving means which moves the above-described suction nozzle 8 relatively to the above-described accommodating container 5 and culture container 3; and inspection means 10 that checks the cultured state of the cell mass sheet 4 in the above-described culture container 3 (see FIG. 6).

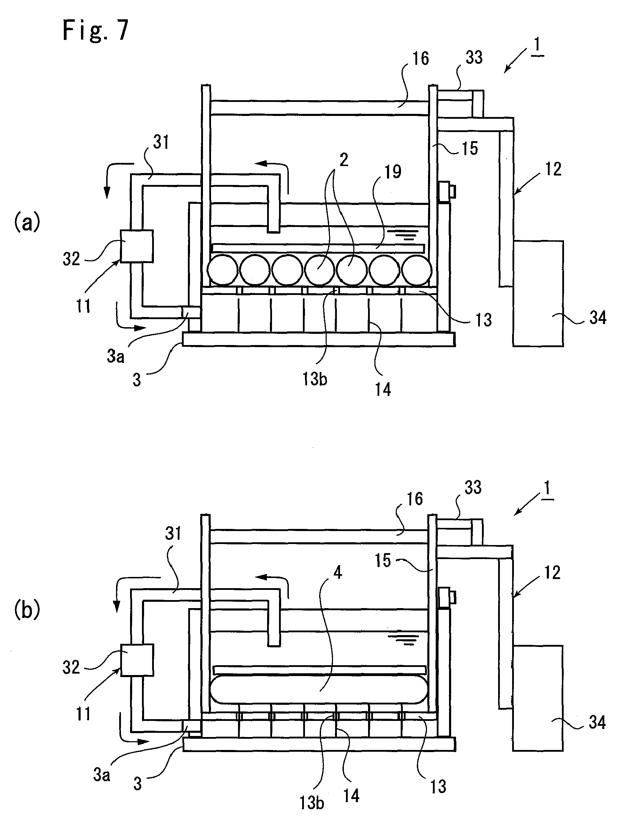

On the other hand, FIG. 7 shows a structure provided in the inner part of the above-described incubator 62. The structure includes: culture liquid supply means 11 which supplies the culture liquid to the cell masses 2 or the cell mass sheet 4 on the culture container 3; and cell mass sheet moving means 12 which moves the cell mass sheet 4 which is being cultured. The above-described cell mass 2 or cell mass sheet 4 is cultured in the inner part of the incubator 62, and accordingly the structure in the inner part of the incubator 62 constitutes the apparatus for culturing the cell masses or the apparatus for culturing the cell mass sheet.

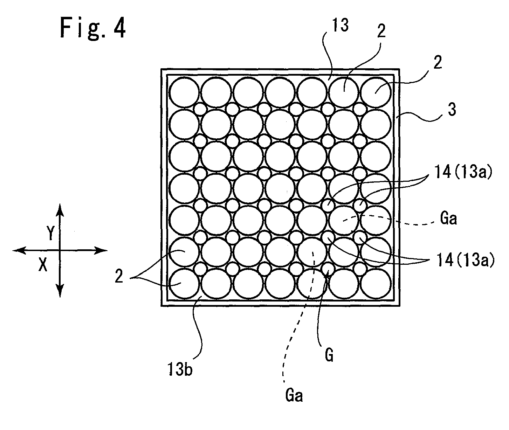

Incidentally, in the following description, a left-right direction is defined as an X direction, a front-rear direction is defined as a Y direction, and an upper-lower direction is defined as a Z direction, in FIG. 1; and in FIG. 4, a left-right direction is defined as the X direction, and an upper-lower direction is defined as the Y direction.

In the above-described isolator 61, the inside is kept in a sterile environment, and a flow of sterile air in one direction, which heads to the lower part from the upper part, is formed by sterile air supply means.

In addition, a glove 61a that an operator can wear is provided on the front face of the isolator 61 so that the operator can perform various works. Incidentally, it is also possible to provide a robot or transfer means having a required structure in the inside of the isolator 61, and make these works automatically performed.

Next, in the above-described incubator 62, the inside is kept in a sterile environment, and also is kept at predetermined temperature and humidity that are suitable for culture of the cell mass sheet 4; and the above-described incubator 62 is structured to be separated from the above-described isolator 61 so that the cell masses 2 can be cultured in a place separated from the isolator 61 while the cell masses 2 are fused to each other by being cultured and are formed into the cell mass sheet 4.

Because of this, the above-described isolator 61 and incubator 62 are connected to each other by connection means 64, and connection means can be used that contacts/separates the incubator 62 with/from the isolator 61 while the sterile condition is kept, as is described, for instance, in Japanese Patent No. 4656485.

FIG. 2 shows a cross-sectional view of the above-described accommodating container 5; and the accommodating container 5 has a plurality of recessed accommodating portions 5a provided lengthwise and breadthwise when being viewed as the plane, and the cell masses 2 each having an approximately spherical shape are accommodated in the inside of the recessed accommodating portions 5a together with a culture liquid, one by one respectively.

A predetermined number of the recessed accommodating portions 5a in the above-described accommodating container 5 are arrayed each in the X-direction and the Y-direction when being viewed as the plane, and in the present embodiment, the same number as the number of the cell masses 2 that constitute the cell mass sheet 4 which is produced in the culture container 3, in the X-direction and the Y-direction, specifically, seven recessed accommodating portions 5a in the X direction and seven recessed accommodating portions 5a in the Y direction are provided, respectively.

The accommodating container supporting section 6 which supports the above-described accommodating container 5 positions the above-described accommodating container 5 on the upper face thereof by a positioning piece 6b, and also is formed of a Y-direction table 6a which constitutes the above-described nozzle moving means 9.

As will be described later, in the present embodiment, the seven suction nozzles 8 are provided in the X direction and are structured so as to be capable of moving only in the X direction and the Z direction; and accordingly, the suction nozzles 8 and the accommodating container 5 can be relatively moved in each of the directions of X, Y and Z, by causing the above-described Y-direction table 6a to move the accommodating container 5 in the Y-direction.

The above description will be specifically described below. The above-described Y-direction table 6a is structured so as to firstly position the recessed accommodating portions 5a of the first row of the accommodating container 5 below the suction nozzles 8, and when the suction nozzles 8 adsorb the cell masses 2 from the recessed accommodating portions 5a of the first row and hold those thereon, move the accommodating container 5 in the Y-direction by one row to position the recessed accommodating portions 5a of the second row below the suction nozzles 8.

As is shown in FIG. 1, a culture tray 13 is accommodated so as to be capable of moving up and down in the above-described culture container 3, and the culture container 3 is structured to be supported by the above-described culture container supporting section 7.

As is shown in FIG. 3, the above-described culture tray 13 has an approximately square plate shape, and as is shown in FIG. 4, the upper face thereof functions as a mounting surface 13a and is set at a size in which the seven cell masses 2 can be arrayed each in the X-direction and the Y-direction in a state of coming in contact with each other.

The mounting surface 13a of the culture tray 13, on which the cell masses 2 are arranged, has non-adhesive properties for cells, and is formed so as to be difficult for the cell masses 2 and the cell mass sheet 4 to adhere to. The cell masses 2 resists adhering to the mounting surface 13a, accordingly, an action for the adjacent cell masses 2 to tend to be connected to each other becomes strong, and the formation of the cell mass sheet 4 is promoted.

Incidentally, the non-adhesiveness to cells is obtained by an operation of making the properties of the surface water-repellent or super-hydrophilic, and can be achieved by adopting a known material having these properties, or subjecting the surface to surface treatment or coating treatment. In addition, it is possible to form a DLC (diamond-like carbon) film, as a method of achieving the surface properties of the mounting surface 13a.

Furthermore, the above-described culture tray 13 is formed from a material which allows light to pass therethrough, and when a coating film or the like is formed on the above-described mounting surface 13a, the coating film also allows the light to pass therethrough.

The above-described culture container 3 has a closed-end box shape, accommodates the culture liquid therein up to a predetermined depth, and has a plurality of pins 14 regularly provided on the bottom face thereof, which function as guide means G.

The bottom face of the culture container 3 is formed into an approximately same shape as that of the above-described culture tray 13, and thereby the cell masses 2 positioned in the outer peripheral side, among the cell masses 2 which have been arrayed on the mounting surface 13a of the above-described culture tray 13, come in contact with the inner wall of the culture container 3; and thereby is structured so as to support the arrayed cell masses 2 from the outside so that the cell masses 2 do not move.

In addition, the culture container 3 is formed of a transparent resin or glass, can allow the light pass therethrough, and is enabled to make the whole culture container 3 inspected in the above-described inspection means 10.

The culture tray 13 is structured so as to be positioned at a lowered position at which the culture tray 13 comes in contact with the bottom face of the culture container 3, and at a raised position at which the culture tray 13 has risen from the lowered position.

Columns 15 are provided on four corners of the above-described culture tray 13, and the adjacent columns 15 are connected to each other by a reinforcing member 16 that is provided on the upper part of the columns 15. The columns 15 are allowed to move only in the upper-lower direction (Z-direction) along the inner surface of the above-described culture container 3, and because of this, the culture tray 13 is structured so as to be capable of moving up and down in the inside of the above-described culture container 3 while the horizontal state is kept.

On the other hand, on the above-described culture container 3, a locking mechanism 17 is provided that is provided at a position through which any of the columns 15 passes, and appears and disappears by an action of a not-shown spring or the like.

In the column 15 that passes through the above-described locking mechanism 17, among the above-described columns 15, a recessed portion 15a is formed with which the above-described locking mechanism 17 is engaged, and is structured so that when the above-described culture tray 13 is positioned at the raised position, the above-described locking mechanism 17 projects, is engaged with the above-described recessed portion 15a, and holds the culture tray 13 at the raised position.

On the other hand, when the culture tray 13 is vertically moved from this raised position, a predetermined force may be exerted on the above-described column 15 in the Z-direction. The above-described locking mechanism 17 is structured to be thereby recessed by resisting a pressing force of a spring, and be separated from the above-described recessed portion 15a.

The above-described pins 14 are fixed on the bottom face of the culture container 3 so as to be oriented upward, and a plurality of through holes 13b are bored in the above-described culture tray 13 so as to match the positions of the above-described pins 14.

Accordingly, when the above-described culture tray 13 is positioned at the lowered position, the above-described pins 14 pass through the above-described through holes 13b and project to the upside of the culture tray 13, and the guide means G becomes a state of being placed above the culture tray 13.

On the other hand, when the culture tray 13 is positioned at the raised position, the culture tray 13 is positioned above the tip portions of the above-described pins 14 and the pins 14 are separated from the through holes 13b, and accordingly, the guide means G becomes a state of having retracted from the mounting surface 13a of the above-described culture tray 13.

Next, as is shown in FIG. 4, in the present embodiment, seven cell masses 2 are arrayed each in the X-direction and the Y-direction lengthwise and breadthwise on the culture tray 13, and the above-described pins 14 are provided in the periphery of each of the cell masses 2; and a space that has been formed by the plurality of pins 14 and pins 14 is structured to constitute an accommodating portion Ga which accommodates each of the cell masses 2 therein.

Specifically, the pins are structured so that the four pins 14 surround the periphery of one cell mass 2 and a space between the adjacent pin 14 and pin 14 becomes narrower than a diameter of the above-described cell mass 2.

As a result, the cell mass 2 can be accommodated in the above-described accommodating portion Ga, and thereby the cell masses 2 which are accommodated in the adjacent accommodating portions Ga can be brought into contact with each other. The space and the degree of contact between the cell masses 2 at this time can be adjusted by a distance between the pin 14 and the pin 14, and the diameter of the pin 14.

In addition, in the present embodiment, in the case where the above-described cell masses 2 come in contact with the wall surface of the above-described culture container 3, when the cell masses 2 have been arrayed on the mounting surface 13a of the culture tray 13, the above-described accommodating portion Ga results in being formed also by the wall surface and the pin 14.

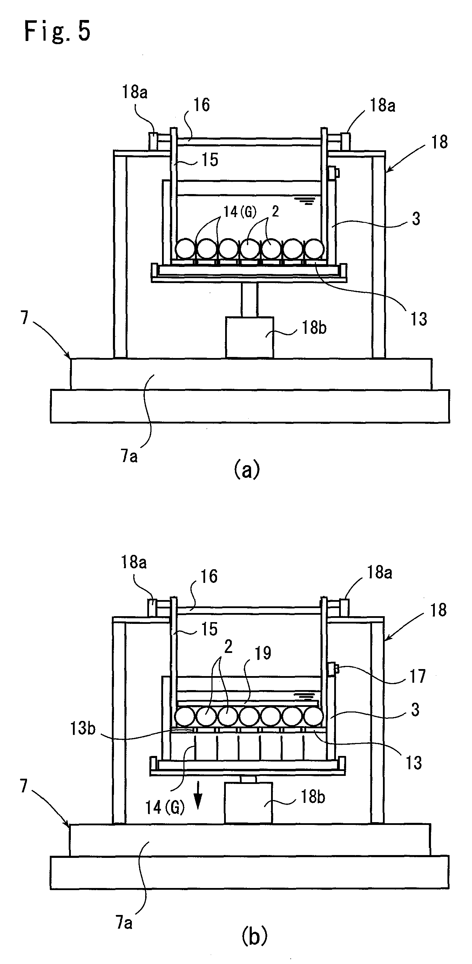

As is shown in FIG. 5, the culture container supporting section 7 includes: a guide moving means 18 that moves the above-described guide means G; and a Y-direction table 7a which constitutes the above-described nozzle moving means 9.

The above-described guide moving means 18 includes: holding means 18a that holds the reinforcing member 16 of the column 15 which is provided on the culture tray 13 and holds the above-described culture tray 13 at a predetermined height; and elevating means 18b which supports the above-described culture container 3 and moves the culture container 3 up and down.

The above-described elevating means 18b moves the culture container 3 up and down in a state in which the above-described holding means 18a holds the above-described culture tray 13, thereby relatively moves the culture container 3 and the culture tray 13 up and down, and can make the culture tray 3 positioned at the lowered position and the raised position.

The above-described Y-direction table 7a is structured so as to move the whole culture container 3 that contains the accommodating portions Ga on the culture tray 13, in the above-described Y-direction one row by one row, every time the cell masses 2 that have been adsorbed and held by the suction nozzles 8 are accommodated in the above-described accommodating portions Ga on the culture tray 13, similarly to the Y-direction table 6a that constitutes the above-described accommodating container supporting section 6.

Furthermore, at a position which is adjacent to the above-described culture container supporting section 7, pressing lid supply means is provided that is not shown and supplies a pressing lid 19 which acts as floating preventing means, onto the upper part of the cell masses 2 that have been arrayed on the above-described culture tray 13.

A robot or the like can be used as the pressing lid supply means, and the pressing lid supply means is structured so as to supply the above-described pressing lid 19 onto the upper part of the arrayed cell masses 2, when the cell masses 2 have been arrayed on the above-described culture tray 13.

The above-described pressing lid 19 is formed of a member that has a mesh form or has a large number of through holes formed therein, and is structured so as not to hinder the culture liquid from being supplied to the cell masses 2 and the cell mass sheet 4 on the culture tray 13.

In addition, the pressing lid 19 is formed from a material which allows the light to pass therethrough, similarly to the culture tray 13, and a side in the pressing lid 19, which comes in contact with the cell masses 2 or the cell mass sheet 4, becomes non-adhesive to cells, similarly to the mounting surface 13a of the above-described culture tray 13.

Next, the suction nozzle 8 which acts as the above-described holding means will be described. As is shown in FIG. 2, the suction nozzle 8 has a main body portion 8a that is connected to not-shown negative pressure generating means, and an adsorbing portion 8b that has a tubular shape and is provided beneath the bottom end of the main body portion 8a.

The inner diameter of the above-described adsorbing portion 8b becomes a smaller diameter than that of the above-described cell mass 2, and is formed so that the cell mass 2 is not sucked into the inside of the adsorbing portion 8b, when the tip of the adsorbing portion 8b has adsorbed and held the cell mass 2 thereon, in the recessed accommodating portion 5a of the above-described accommodating container 5.

In addition, above the mounting surface 13a of the culture tray 13, the nozzle moving means makes the central position of the above-described adsorbing portion 8b positioned in the approximately center of the accommodating portion Ga that is formed by the pin 14 and the pin 14, and in this state, the above-described negative pressure generating means cancels the negative pressure. Thereby, the cell mass 2 can be arranged in the accommodating portion Ga.

Incidentally, the holding means may be the one which has such a structure as to hold the cell mass 2 with a gripper or the like, instead of the suction nozzle 8 which adsorbs and holds the cell mass 2 thereon.

In addition, the nozzle moving means 9 that acts as moving means which moves the above-described suction nozzles 8 includes: an X-direction rail 21 that is provided in the X-direction from the above-described accommodating container supporting section 6 to the above-described culture container supporting section 7; X-direction moving means 22 that moves the suction nozzles 8 in the X-direction along the X-direction rail 21; Z-direction moving means 23 that is provided on the X-direction moving means 22 and moves the suction nozzles 8 up and down in the Z-direction; and the above-described Y-direction tables 6a and 7a on the above-described accommodating container supporting section 6 and the above-described culture container supporting section 7. Then, the holding means such as the suction nozzles 8 and the moving means thereof constitute supply means of the cell masses 2.

In addition, the seven suction nozzles 8 in the present embodiment are provided in the X-direction in the state of being arrayed, and are structured so that the spaces can be changed in the X-direction by space changing means 24.

Specifically, when the above-described suction nozzles 8 are positioned above the accommodating container supporting section 6, the space changing means 24 changes the spaces between the above-described suction nozzles 8 to the same spaces as those between the recessed accommodating portions 5a that are arrayed in the X-direction in the above-described accommodating container 5.

On the other hand, when the suction nozzles 8 are positioned above the culture container supporting section 7, the space changing means 24 changes the spaces between the suction nozzles 8 to the same spaces as the spaces in the X-direction between the accommodating portions Ga that are formed by the pins 14 which project to the upside of the mounting surface 13a.

Incidentally, it is also possible to provide a plurality of rows of the suction nozzles 8 in the Y-direction, and when the spaces between the recessed accommodating portions 5a on the accommodating container supporting section 6 are the same as the spaces between the accommodating portions Ga that are formed on the culture tray 3, the above-described space changing means 24 can be omitted.

In addition, for instance, when the spaces between the above-described accommodating portions Ga are narrow, it is also possible to accommodate the cell masses 2 in every other accommodating portions Ga by the above-described suction nozzles 8, and in this case, firstly, it is acceptable to mount the cell masses 2 in the odd-numbered accommodating portions Ga on the mounting surface 13a, and then to mount the cell masses 2 in the even-numbered accommodating portions Ga.

Incidentally, at least one row of suction nozzles 8 may be provided, and the supply means of the cell masses 2 may have, in addition to a structure in which the suction nozzles move and mount the cell masses 2 as in the present embodiment, such a structure as to send out the cell masses 2 one by one from the container in which a large number of cell masses 2 are accommodated.

In addition, on the above-described X-direction rail 21, a camera 25 is provided that moves in the X-direction, and the above-described camera 25 is structured so as to move to the upper part of the culture container supporting section 7, and to photograph the mounting surface 13a, when the cell masses 2 have been arranged on the culture tray 13 by the above-described suction nozzles 8.

It is checked whether or not the cell masses 2 are arranged in all of the accommodating portions Ga on the above-described mounting surface 13a by the photographed image, and the operator can direct the accommodating portions Ga which do not have the cell masses 2 accommodated therein, so as to accommodate the cell masses 2, as needed.

In addition, the camera 25 is structured, when the above-described nozzle moving means 9 makes the suction nozzles 8 positioned above the culture container supporting section 7, so as to retract from the upper part of the culture container supporting section 7, in order to avoid the contact with the suction nozzle 8.

The inspection means 10 shown in FIG. 6 has illumination means 26 that is provided in the lower part, and a camera 27 for inspection that is provided in the upper part, and the above-described culture container 3 is structured so as to be held between the illumination means 26 and the camera 27 for inspection, by a not-shown robot or other means.

As has been described above, the above-described culture tray 13, the bottom part of the culture container 3, and the pressing lid 19 are each formed from the optically transparent material, and accordingly, the light that the above-described illumination means 26 has emitted passes through the culture tray 13 and the bottom part of the culture container 3, passes through the cell masses 2 or the cell mass sheet 4 on the culture tray 13, and is received by the camera 27 for inspection.

The above-described camera 27 for inspection can check these cultured states on the basis of the photographed cell mass 2 or cell mass sheet 4, and can determine to continue or stop culturing, according to the quality of the cultured states.

Incidentally, in the present embodiment, the above-described illumination means 26 and the camera 27 for inspection are provided in the inside of the isolator 61, but it is also acceptable to make a part of the above-described isolator 61 project to the outside, also to form the upper part and the lower part of the projecting portion of the optically transparent members, and to make these members hold the above-described culture container 3 therebetween.

FIG. 7 shows the culture liquid supply means 11 that supplies the culture liquid to the above-described cell mass sheet 4, and the cell mass sheet moving means 12 that moves the cell mass sheet 4 that is being cultured on the above-described culture tray 13, which are provided in the inside of the incubator 62.

When a plurality of culture containers 3 are accommodated in the inside of the above-described incubator 62, the above-described culture liquid supply means 11 and cell mass sheet moving means 12 can be provided on each of the culture containers 3.

A connection port 3a is provided in the lower part of the above-described culture container 3, and specifically, the connection port 3a is provided so as to be positioned below the culture tray 13, in such a state that the above-described culture tray 13 is positioned at the raised position.

The above-described culture liquid supply means 11 includes: a pipe 31 of which one end is connected to the above-described connection port 3a and the other end is inserted into a liquid level of the culture liquid, from above the above-described culture container 3, and a liquid sending pump 32 that is provided in the pipe 31 and sends the culture liquid.

The above-described liquid sending pump 32 is structured so as to suck the culture liquid from the upper side of the above-described culture container 3, also make the culture liquid flow into the culture tray container 3 from the lower part thereof through the above-described connection port 3a, and thereby make the culture liquid circulate in the culture container 3.

On the other hand, the above-described culture tray 13 is carried into the incubator 62 in a state of being positioned at the raised position in the culture container 3, and because of this, the above-described pins 14 are positioned below the above-described culture tray 13.

In addition, through holes 13b which vertically penetrate the culture tray 13 are bored so as to match the positions of the above-described pins 14, and specifically are bored at positions between the above-described cell mass 2 and cell mass 2 that have been arrayed on the mounting surface 13a of the above-described culture tray 13, and make the mounting surface 13a side of the culture tray 13 communicate with the lower face side thereof.

Due to such a structure, the culture liquid which has been supplied by the above-described culture liquid supply means 11 passes through the above-described through holes 13b, and is supplied from the lower face side of the culture tray 13 to the mounting surface 13a side, specifically to a space between the mounting surface 13a and the back face side of the above-described cell mass sheet 4; and the above-described through holes 13b can be used as supply holes of the culture liquid.

As a result, the structure can supply the culture liquid to the back face side of the cell mass sheet 4, which comes in contact with the culture tray 13, can bring the culture liquid into contact with the back face side, and can adequately culture the back face side of the cell mass sheet 4, to which the culture liquid tends to be insufficiently supplied because the back face side comes in contact with the culture tray 13.

Incidentally, when a liquid-sending direction of the liquid sending pump 32 is reversed, the culture liquid is sucked from the back face side of the cell mass sheet 4 through the through holes 13b; and accordingly, the culture liquid flows into the back face side from the periphery of the cell mass sheet 4, and the culture liquid can be supplied. In this case, the through holes 13b function as discharge holes.

As for the above-described cell mass sheet moving means 12, the through holes 13b that are bored in the above-described culture tray 13, and the pins 14 that are provided on the above-described culture container 3 and penetrates the above-described through holes 13b can be used.

In addition, in the incubator, a holding member 33 that holds the reinforcing member 16 of the column 15 which is provided on the above-described culture tray 13, and elevating means 34 that moves the holding member 33 up and down are provided; and the culture tray 13 is structured so as to be moved up and down in the above-described culture container 3, by the holding member 33 which is moved up and down by the above-described elevating means 34.

As is shown in FIG. 7(B), the pins 14 are structured, when the above-described elevating means 34 moves the culture tray 13 down and the above-described pins 14 penetrate the culture tray 13 from the lower part, so as to slightly project from the culture tray 13 and push up the cell mass sheet 4 from the lower part.

Here, the mounting surface 13a of the culture tray 3 is formed so as to be non-adhesive, and accordingly, the cell mass sheet 4 can be easily peeled off from the mounting surface 13a by the above-described pins 14.

Furthermore, the above-described cell mass sheet moving means 12 operates at predetermined intervals. Due to such an action, the cell mass sheet 4 can be easily collected from the culture container 3 when the culture has ended, and in addition, it can be expected that the action gives stimulus to the cell masses 2 and activates the cell masses 2 which are being cultured.

Incidentally, the pins 14 do not need to be completely pulled out from the through holes 13b.

A method for producing the cell mass sheet 4 with the use of the apparatus 1 for producing the cell mass sheet, which has the above-described structure, will be described below.

Firstly, a preparation step is performed that carries the accommodating container 5 in which the above-described cell masses 2 are accommodated and the culture container 3 in which the above-described culture tray 13 is accommodated, into the inside of the above-described isolator 61 through the above-described pass box 63, and supplies a predetermined amount of the culture liquid to the culture container 3.

At this time, if a plurality of above-described accommodating containers 5 and culture containers 3 are carried into the inside of the isolator 61, it becomes possible to continuously produce a plurality of cell mass sheets 4 by using the plurality of culture containers 3.

Next, a guide placing step is performed that places the pins 14 which act as the above-described guide means G, on the mounting surface 13a of the above-described culture tray 13. Specifically, on the above-described culture container supporting section 7, the above-described guide moving means 18 makes the culture tray 13 positioned at the lowered position, and thereby the above-described pins 14 project to the upper face of the culture tray 13, and the guide means G that has the accommodating portion Ga formed therein results in being placed on the mounting surface 13a.

Incidentally, this guide placing step can be performed simultaneously with the above-described preparation step, if the culture container 3 in which the culture tray 13 has been previously positioned at the lowered position is carried into the isolator.

Thus, when the guide means G has been placed on the above-described culture tray 13, subsequently, an accommodating step is performed that moves the above-described suction nozzles 8, and accommodates the cell masses 2 in the above-described accommodating portions Ga of the guide means G, respectively.

The above-described nozzle moving means 9 moves the suction nozzles 8, and while the space changing means 24 changes the spaces between the suction nozzles 8, the suction nozzles 8 adsorb and hold the cell masses 2 from the recessed accommodating portions 5a in the accommodating container 5, and accommodate the arrayed cell masses 2 in the accommodating portions Ga of the guide means G that is placed on the culture tray 13 in the culture container 3.

The above-described accommodating portion Ga is formed by four pins 14 that surround the periphery of the cell mass 2, and thus is structured so as to prevent the cell mass 2 that is accommodated in the accommodating portion Ga from flying out, and keep the arrayed state of the cell masses 2.

Thus, when the cell masses 2 have been accommodated in all of the accommodating portions Ga, the above-described camera 25 moves to the upper part of the culture container supporting section 7, photographs the cell masses 2 on the mounting surface 13a, and checks whether or not the cell masses 2 are accommodated in all of the accommodating portions Ga.

Subsequently, a floating preventing step is performed which arranges the pressing lid 19 on the upper part of the cell masses 2 that are arrayed on the culture tray 13.

Specifically, the not-shown pressing lid supply means or the operator who wears the glove 61a charges the pressing lid 19 into the inside of the above-described culture container 3, and prevents a lift of the cell masses 2 on the mounting surface 13a, by the pressing lid 19.

Next, a guide retracting step is performed which retracts the pins 14 that act as the guide means G, from the mounting surface 13a of the above-described culture tray 13, from a state in which the cell masses 2 are accommodated in the accommodating portions Ga of the above-described guide means G.

Specifically, the culture tray 13 is positioned at the raised position with respect to the culture container 3 by the above-described guide moving means 18 of the culture container supporting section 7, and thereby the pins 14 that have projected from the upper face of the above-described mounting surface 13a are retracted from the mounting surface 13a.

In addition, the adjacent cell masses 2 are brought into contact with each other when having been accommodated in the above-described accommodating portions Ga, and accordingly, the cell masses 2 which are arrayed lengthwise and breadthwise result in remaining on the mounting surface 13a while keeping the state in which the cell masses have been brought into contact with each other.

Thus, when the cell masses 2 have been arrayed on the culture tray 13, the whole culture container 3 that contains the above-described culture tray 13 is transferred to the incubator 62, by the robot or the manual work of the operator, and is subjected to a culture step that cultures the cell masses 2 which are arrayed on the culture tray 13.

Firstly, the above-described culture tray 13 is transferred to the incubator 62; and then the incubator 62 is separated from the above-described isolator 61, and is mounted on a position that is separated from the isolator 61.

Then, the inside of the incubator 62 is kept at a predetermined temperature and a predetermined humidity, predetermined concentrations of carbon dioxide and oxygen are kept, and the cell masses 2 on the culture tray 13 are cultured. Thereby, the adjacent cell masses 2 are fused to each other, and finally, one cell mass sheet 4 results in being formed.

At this time, the cell masses 2 keep a state of being arrayed on the mounting surface 13a of the culture tray 13 and having been brought into contact with each other; and accordingly, the formed cell mass sheet 4 results in having approximately the same shape as the shape of the mounting surface 13a of the above-described culture tray 13, and the thickness thereof can be approximately uniformized.

During the above-described culture step, two steps are performed in the incubator 62, one of which is a culture liquid supply step of supplying the culture liquid to the cell masses 2 or the cell mass sheet 4 on the culture tray 13 at predetermined intervals, and another one of which is a cell mass sheet moving step of moving the cell mass sheet 4 on the culture tray 13.

As for the above-described culture liquid supply step, the culture liquid supply means 11 is determined to operate at the predetermined intervals; and is structured to generate a flow of the culture liquid from the lower part to the upper part in the culture container 3, and thereby to supply a new culture liquid to the cell masses 2 or the cell mass sheet 4.

In addition, the culture tray 13 is positioned at the raised position in the culture container 3, and the through holes 13b are bored that act as the above-described supply holes; and accordingly, the culture liquid can be supplied to a space between the cell mass sheet 4 and the mounting surface 13a through the through holes 13b, and the back face side of the cell mass sheet 4 can be efficiently cultured.

In other words, when the cell masses 2 are arrayed and the cell mass sheet 4 is produced as in the present embodiment, there has been a problem in that the culture liquid does not sufficiently spread to the back face side of the cell masses 2 which are positioned in the middle, but by performing this culture liquid supply step, it has become possible to adequately culture also the cells which are positioned in the portion.

Then, when the adjacent cell masses 2 have been fused to each other and the cell mass sheet 4 has been formed, the culture tray 13 that has been positioned at the raised position is moved down by the above-described elevating means 34, as the above-described cell mass sheet moving step.

Then, the above-described pins 14 project to the upper face of the culture tray 3, the cell mass sheet 4 is pushed up from the lower part by the pins 14, and the cell mass sheet 4 is separated from the culture tray 13. At this time, because the surface of the culture tray 13 is formed so as to be non-adhesive, it is possible to separate the cell mass sheet 4 and move the cell mass sheet 4 upward, with a little pressing force.

This cell mass sheet moving step is performed at predetermined intervals; and thereby either of such states can be kept that the cell mass sheet 4 does not adhere to the culture tray 13 or that the cell mass sheet 4 can be peeled from the culture tray 13 with a smaller force, and moderate stimulus can be given to the cell mass sheet 4.

When the cell mass sheet 4 has been formed to some extent in the above-described culture step, an inspection step is performed that connects the incubator 62 to the isolator 61 at fixed periods, and checks the cultured state of the cell mass sheet 4.

In the inspection step, the culture container 3 is transferred to the isolator 61 from the connected incubator 62, and is further transferred to the above-described inspection means 10 shown in FIG. 6.

In the inspection means 10, the light that the illumination means 26 has emitted from the lower part passes through the above-described culture container 3, culture tray 13, cell mass sheet 4 and pressing lid 19, and the quality of the culture of the cell mass sheet 4 is inspected on the basis of the video image that the camera 27 for inspection has taken.

Then, when it has been determined in the inspection step that the cell mass sheet 4 has been sufficiently cultured, a collection step is subsequently performed that collects the cell mass sheet 4 from the above-described culture container 3.

By performing the above-described cell mass sheet operation step during the above-described culture step, it is possible in the collection step to peel the cell mass sheet 4 from the mounting surface 13a without using a chemical agent such as trypsin and collect the cell mass sheet 4, and to suppress damage to the cells to a minimum.

Incidentally, the guide retracting step in the above-described embodiment is performed before the culture tray 3 is transferred to the above-described incubator 62, but may be performed after the culture tray 3 has been transferred to the incubator 62.

Specifically, it is possible to perform the above-described culture step in a state in which the pins 14 that act as the above-described guide means G project to the inside of the mounting surface 13a of the culture tray 13, and after the adjacent cell masses 2 have been fused to each other and the cell mass sheet 4 has been formed, to pull the pins 14 from the formed cell mass sheet 4. In addition, it is also acceptable to perform the guide retracting step after the cell masses 2 have been cultured to some extent, and then to continue the culture.

Furthermore, in the culture liquid supply step in the above-described embodiment, it is also acceptable to push or pull the pins 14 into or from the through holes 13b by vertically moving the above-described culture tray 13 while the above-described culture step is performed, thereby push or pull the culture liquid into or from the through holes 13b by the action of the pins 14 to distribute the culture liquid, and supply the culture liquid to the back face side of the cell mass sheet 4.

In addition, in place of the cell mass sheet moving step in the above-described embodiment, it is also acceptable to make the above-described culture liquid supply means 11 supply the culture liquid with a predetermined pressure, spout the culture liquid toward the upper part from the through hole 13b of the above-described culture tray 13, and thereby push up the cell mass sheet 4 from the lower part.

In this case, the holding member 33 and the elevating means 34 which act as the above-described cell mass sheet moving means 12 can be omitted, and the above-described culture liquid supply means 11 can be used as the cell mass sheet moving means 12.

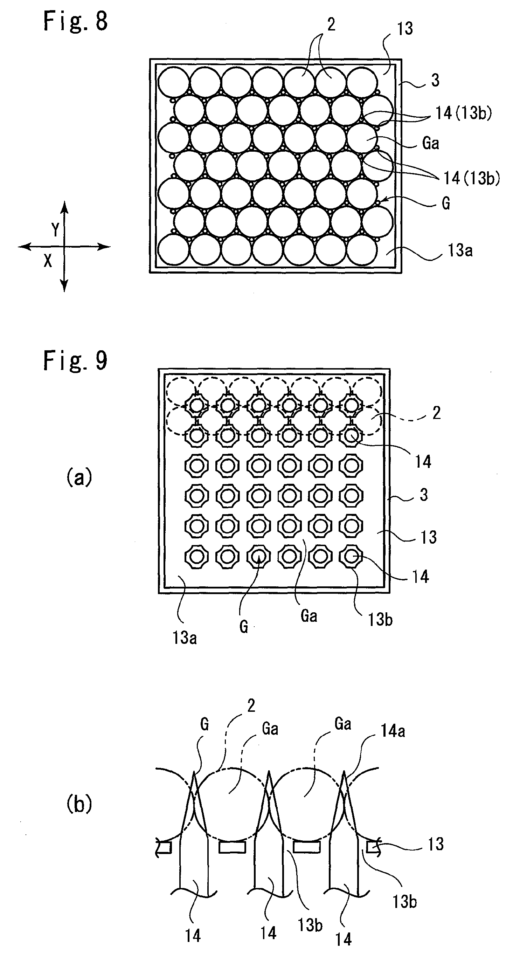

FIG. 8 is a view for describing a variation of the culture tray 13 and the pins 14 that act as the guide means G, which can be used in the apparatus 1 for producing the cell mass sheet according to the above-described first embodiment.

The above-described cell masses 2 are mounted in a staggered form on the culture tray 13 shown in FIG. 8; and specifically, the cell masses 2 are linearly arrayed in the X-direction, and are arrayed in the Y-direction while being shifted by a half piece from each other.

In order to thus array the cell masses 2, the through holes 13b that are bored in the above-described culture tray 13 and the pins 14 that act as the guide means G are arranged so that six pins 14 are positioned in the periphery of each of the cell masses 2 to form the accommodating portion Ga.

Also in this culture tray 13, the cell masses 2 are arranged so as to come in contact with each other between the adjacent pin 14 and pin 14, and even when the pins 14 have been removed, the state in which the cell masses 2 come in contact with each other can be kept, similarly to the state in the above-described embodiment.

Incidentally, the way of arranging the above-described through holes 13b and pins 14 is not limited to the arrangement in the culture tray 13 shown in FIG. 4 and FIG. 8, but the cell masses 2 can be arrayed in various modes, for instance, such as the way of concentrically arranging the cell masses 2 on the circular culture tray 13, and the like.

FIG. 9 is also a view for describing a variation of the culture tray 13 and the pins 14 that act as the guide means G, which can be used in the apparatus 1 for producing the cell mass sheet according to the above-described first embodiment.

On the culture tray 13 in the present embodiment, as shown in FIG. 9(A), four pins 14 are positioned in the periphery of each of the cell masses 2, and the cell masses 2 are arrayed lengthwise and breadthwise, similarly to the above-described first embodiment.

The above-described pin 14 has a larger diameter than that of the pin 14 shown in FIG. 4. In addition, the above-described through hole 13b is formed so as to have a larger diameter than that of the pin 14, and a clearance is formed between the pin 14 and the opening of the through hole 13b. In addition, on four corners of the through hole 13b, four arcuate releasing portions are formed, respectively, so as to match the shape of the cell mass 2.

By thus increasing the size of the through hole 13b so that the clearance is formed between the pin 14 and the opening of the through hole 13b, it becomes possible for the through hole 13b to supply a more culture liquid to the back face side of the cell masses 2 or the cell mass sheet 4, from the through holes 3a that act as the above-described supply holes, in the culture liquid supply step.

On the other hand, as is shown in FIG. 9(B), a tapered portion 14a is formed on the tip portion of the above-described pin 14, and is structured so that the contact between the cell masses 2 is not hindered in a space between the pin 14 and the pin 14 by the tapered portion 14a.

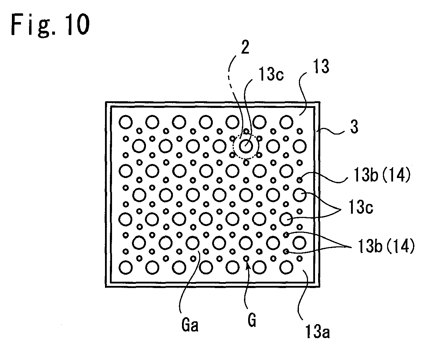

FIG. 10 is also a view for describing a variation of the culture tray 13 which can be used in the apparatus 1 for producing the cell mass sheet according to the above-described first embodiment, and here, the variation will be described with reference to the culture tray 13 used in FIG. 8.

In the culture tray 13 in the present embodiment, a supply hole 13c is provided at a position corresponding to the above-described accommodating portion Ga, specifically, at a position on which the cell masses 2 are mounted, in addition to the through hole 13b that the above-described pin 14 penetrates.

That is to say, in the above-described culture liquid supply step, it becomes possible to supply the culture liquid to the back face side of the cell mass sheet 4 also from the above-described through hole 13b and the above-described supply hole 13c, and to distribute a lot of culture liquid.

In any of the above-described embodiments, the pins 14 are not arranged on the outer peripheral side of the arrayed cell masses 2, but the pins 14 can be arranged also on the outer peripheral side so that the cell masses 2 do not come in contact with the inner wall of the culture container 3.

In this case, the pins 14 in the outer peripheral side are structured so as to remain on the mounting surface 13a, even if the pins 14 except for the pins 14 in the outer peripheral side have been removed from the mounting surface 13a in the guide means retracting step.

More specifically, the pins 14 in the outer peripheral side are formed to be longer than the other pins 14 so that when the culture tray 13 has been positioned at the raised position with respect to the culture container 3 by the above-described guide moving means 18, the pin 14 in the outer peripheral side keeps a state of projecting from the mounting surface 13a. The raised position may be set at two stages so that when the pin 14 in the outer peripheral side also needs to be retracted, the culture tray 13 can be further raised from the above-described raised position.

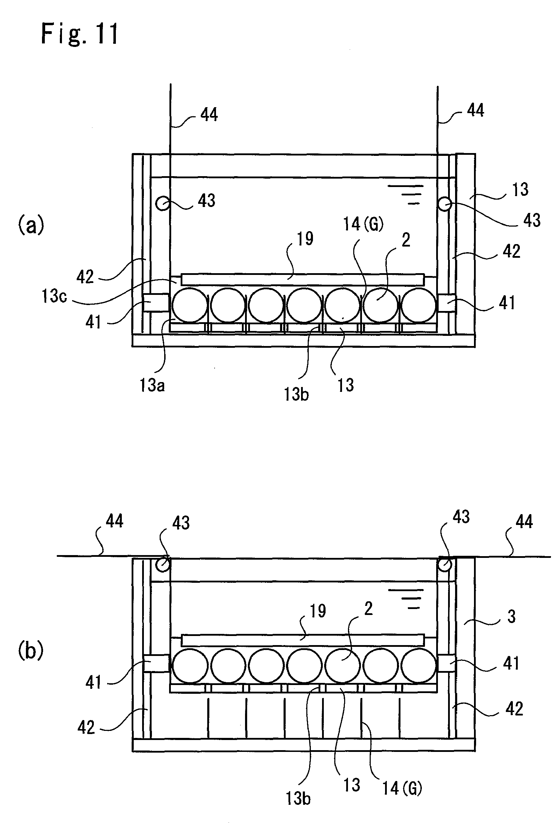

FIG. 11 is also a view for describing a variation of the culture tray 13 and the culture container 3 that can be used in the apparatus 1 for producing the cell mass sheet according to the above-described first embodiment.

The culture tray 13 in the present embodiment has a closed-end box shape, has the mounting surface 13a on which the cell masses 2 are mounted and side faces 13c that are provided so as to be adjacent to the mounting surface 13a, and is structured so as to support the cell masses 2 that are arrayed by the side faces 13c of the culture tray 13, which is different from the culture tray 13 and the culture container 3 in FIG. 3.

The bottom face of the culture container 3 is formed so as to be a size larger than the above-described culture tray 13, and because of this, a clearance is formed between the side face of the culture tray 13 and the inner wall of the culture container 3.

The above-described clearance has a sliding member 41 that is provided on the side face of the above-described culture tray 13, and a guide rail 42 that is vertically provided on the culture container 3, and thereby the culture tray 13 is structured so as to move up and down in the culture container 3 along this guide rail 42.

The above-described culture tray 13 has a locking member 44 that is swingably provided through a hinge 43, and the locking member 44 is structured so as to fall down toward the outside by the above-described hinge 43, in a state in which the culture tray 13 is positioned at the raised position, and be locked at the upper end of the culture container 3, as is shown in FIG. 11(B).

The above-described guide means G has such a structure as to make the pin 14 that is provided on the bottom face of the culture container 3 penetrate the through hole 13b that is provided in the culture tray 13, and thereby to make the pin 14 project to the upside of the mounting surface 13a of the culture tray 13, similarly to the culture tray 13 and the culture container 3 shown in FIG. 3.

In addition, as for the guide moving means 18 that moves the above-described culture tray 13 in the inside of the culture container 3 and makes the above-described pin 14 project, a robot or the like can be used in place of the holding means 18a and the elevating means 18b in FIG. 5.

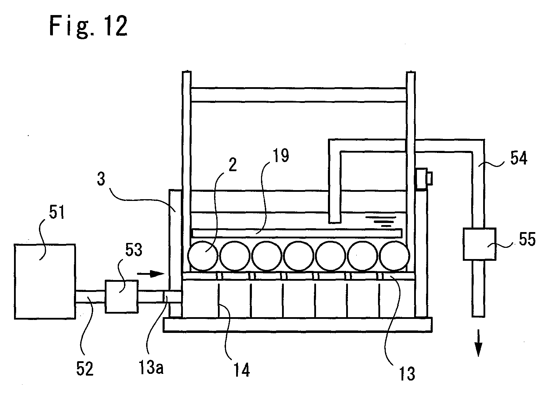

FIG. 12 is a view for describing a variation of the culture liquid supply means 11 that can be used in the apparatus 1 for producing the cell mass sheet according to the above-described first embodiment.

The culture liquid supply means 11 shown in FIG. 7 is structured so as to circulate the culture liquid in the culture container 3 by using the above-described pipe 31 and the liquid sending pump 32 and thereby supply the culture liquid, but the culture liquid supply means 11 in FIG. 12 has such a structure as to be capable of supplying a fresh culture liquid into the above-described culture container 3.

The above description will be specifically described below. The above-described culture liquid supply means 11 includes: a culture liquid tank 51 that stores the fresh culture liquid therein; a supply pipe 52 that is connected between the culture liquid tank 51 and the connection port 3a of the above-described culture container 3; a liquid sending pump 53 that is provided in the supply pipe 52; a discharge pipe 54 of which one end is inserted into the culture liquid of the above-described culture container 3 and the other end is connected to a not-shown liquid discharging tank; and a liquid discharging pump 55 which is provided in the discharge pipe 54.

The above-described structure can distribute the fresh culture liquid into the culture container 3, by supplying the fresh culture liquid of the culture liquid tank 51 to the culture container 3 by the above-described liquid sending pump 53, and discharging the used culture liquid in the culture container 3 by the above-described liquid discharging pump 55.

In this case, it is acceptable to supply the culture liquid to the lower part of the culture tray 13 and to discharge the culture liquid from the upper part as is shown in the figure, but it is also acceptable to supply the culture liquid to the upper part and to discharge the culture liquid from the lower part.

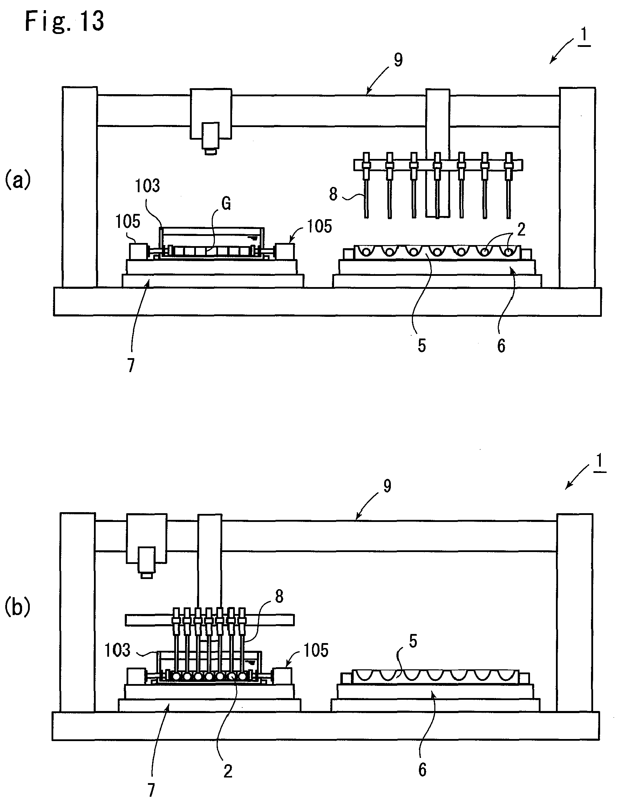

FIG. 13 to FIG. 16 are views for describing an apparatus 1 for producing a cell mass sheet and a method for producing a cell mass sheet 4 according to a second embodiment. Incidentally, the description on the structure which is common to the structure of the first embodiment is omitted, and the common structures are designated by the same reference numerals and will be described.

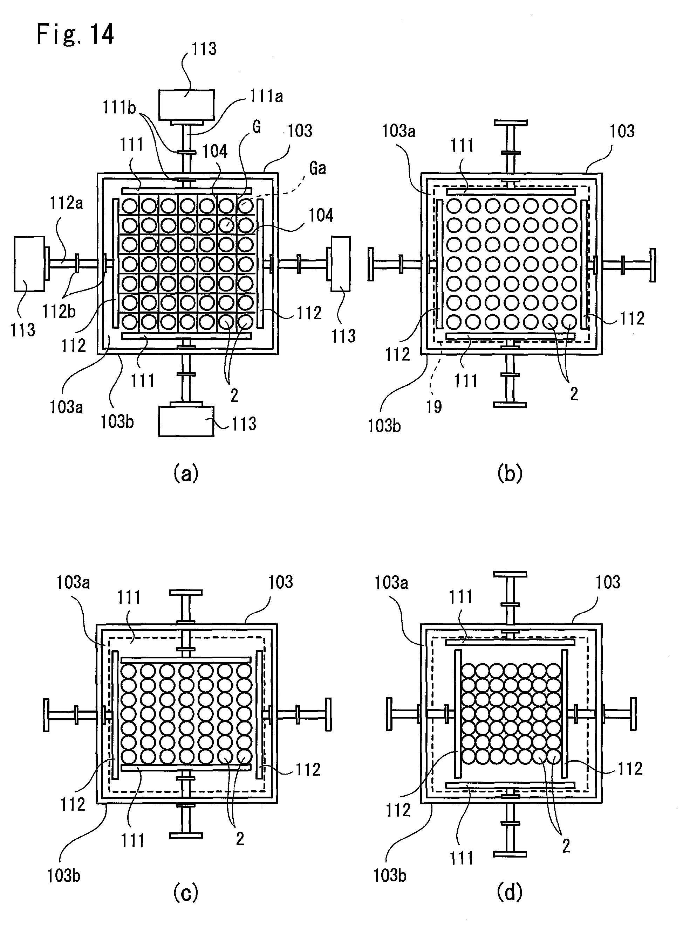

As are shown in FIGS. 13 and 14, a culture container 103 in the present embodiment has a closed-end box shape, also previously accommodates the culture liquid therein, and is structured so as to mount the cell masses 2 on a mounting surface 103a that is the bottom face of the culture container 103.

The guide means G in the present embodiment shown in FIG. 13(A) is structured so as to be mounted on the mounting surface 103a of the above-described culture container 103, and also to be retracted from above the mounting surface 103a while being held.

In addition, the guide means G is formed of partitioning members 104 by which a plurality of partitioned accommodating portions Ga are formed, and is structured so as to make the cell masses 2 arrayed lengthwise and breadthwise in the X-direction and the Y-direction, respectively.

Here, the size of each of the accommodating portions Ga is set so as to be slightly larger than a dimension of the outer diameter of the above-described cell mass 2.

After the cell masses 2 have been accommodated in the above-described accommodating portions Ga, the above-described guide means G is retracted from the culture tray 103, and thereby the cell masses 2 can be arranged in a state of being arrayed on the mounting surface 103a of the culture container 103.

Beside the culture container 103 in the present embodiment, pressing means 105 is provided that presses the cell masses 2 which are arrayed on the above-described mounting surface 103a, from a lateral direction, and brings the adjacent cell masses 2 into contact with each other.

The above-described pressing means 105 includes: first pressing members 111 that mutually approach the cell masses 2 which are arrayed on the above-described culture container 103, from first facing directions (Y-direction), and press the cell masses 2 from the Y-direction; second pressing members 112 that mutually approach the cell masses from second facing directions

(X-direction) which are perpendicular to the above-described Y-direction, and press the cell masses 2 to the X-direction; and moving means 113 which are provided on the above-described culture container supporting section 7 and moves the above-described first and second pressing members 111 and 112 forward and backward.

In four side faces 103b of the above-described culture container 103, connecting bars 111a and 112a are slidably provided that penetrate the side face from the outside to the inside, and the above-described first and second pressing members 111 and 112 are provided on respective ends of the connecting bars 111a and 112a, in the inner side of the culture container 103.

Two stopper members 111b and 112b are provided on the above-described connecting bars 111a and 112a so as to sandwich the side faces 103b of the above-described culture container 103, and stroke amounts of the connecting bars 111a and 112a are determined by distances between the stopper members 111b and between the stopper members 112b.

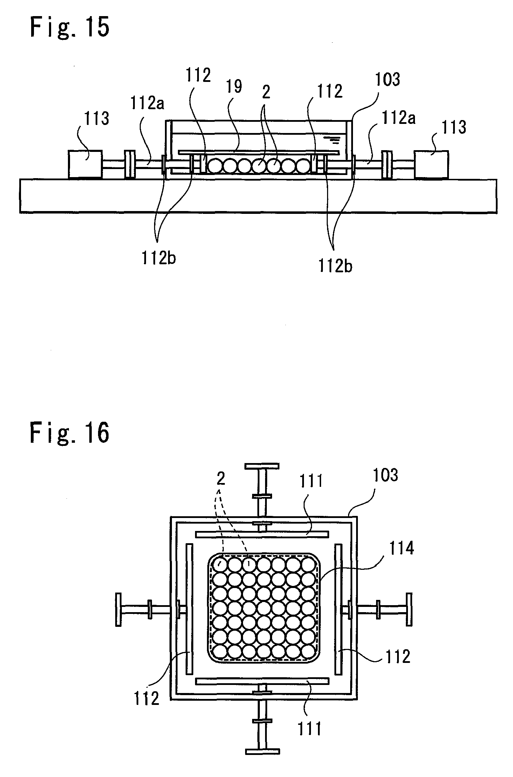

By such a structure, the first and second pressing members 111 and 112 each become reciprocatable to a retreated position (FIG. 14(A)) or to an advanced position (FIG. 14 (C) and (D)). Incidentally, as is shown in FIG. 15, the upper part of the first and second pressing members 111 and 112 is covered with the pressing lid 19, and the pressing lid 19 prevents the cell masses 2 from floating up during a pressing operation.

The distances between the first pressing members 111 and the distances between the second pressing members 112 which are positioned at the above-described retreated position are set so as to be approximately the same as the lengths in the X-direction and the Y-direction of the partitioning members 104 which constitute the above-described guide means G.

When the first pressing members 111 are moved to the advanced position, as is shown in FIG. 14(C), the cell masses 2 are pressed in the Y-direction by the first pressing members 111, and thereby a clearance in the Y-direction between the arrayed cell masses 2 is canceled.

Similarly to the above operation, when the second pressing members 112 are moved to the advanced position, as is shown in FIG. 14(D), the cell masses 2 are pressed in the X-direction by the second pressing member 112, and thereby a clearance in the X-direction between the arrayed cell masses 2 is canceled.

Here, when the second pressing members 112 are moved to the advanced position, in order that the second pressing members 112 avoid contact with the first pressing members 111, the above-described first pressing members 111 are determined to be previously moved to the retreated position.

Thus, the cell masses 2 are pressed from the Y-direction and the X-direction by the first and second pressing members 111 and 112, and thereby the cell masses 2 on the culture container 103 result in being arrayed in a state in which the adjacent cell masses 2 come in contact with each other.

Thus, when the adjacent cell masses 2 are arrayed in a state of coming in contact with each other by the pressing means 105, a holder 114 shown in FIG. 16 is determined to be mounted on the outer edge of the arrayed cell masses 2.

The above-described holder 114 becomes a frame-like member that has been set so as to be approximately the same as the widths of the cell masses 2 which come in contact with each other in the X-direction and the Y-direction, and is structured so as to support the cell masses 2 from the outside so that the arrayed state of the cell masses 2 is not lost.

In addition, on the upper face of the holder 114, a mesh is provided that is structured so as to regulate the lift and movement of the cell masses 2 in the inside of the holder 114, and so as not to hinder the culture of the cell masses 2 in the inside by allowing the distribution of the culture liquid.

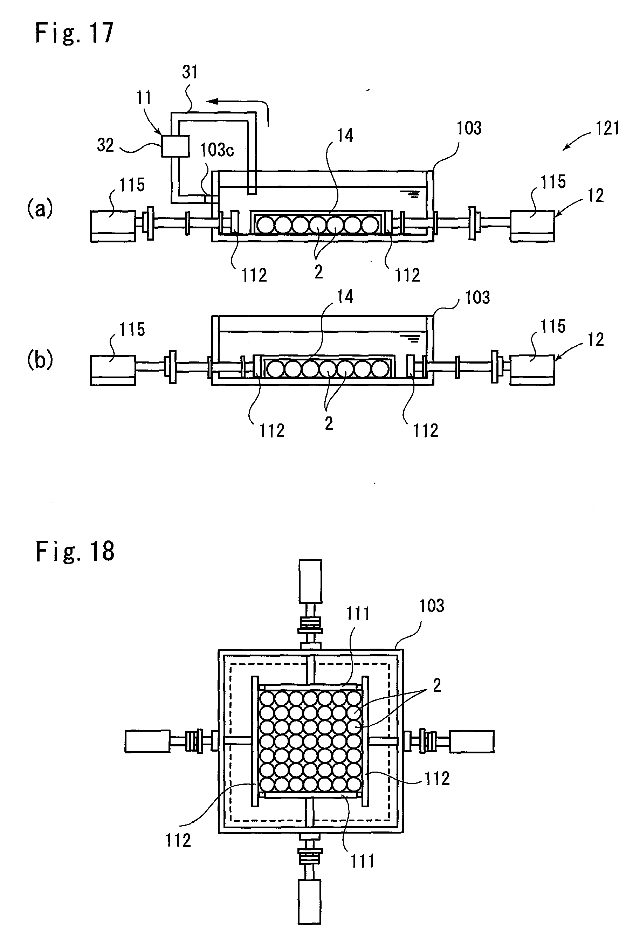

FIG. 17 illustrates cell mass sheet moving means 121 that is provided in the inside of the above-described incubator 62, and that includes: the above-described first and second pressing members 111 and 112 which are provided on the above-described culture tray 103; and moving means 115 which is provided in the inside of the incubator and reciprocatively moves the above-described first and second pressing members 111 and 112.

The above-described first and second pressing members 111 and 112 are structured so as to be reciprocatively moved by the above-described moving means 115, and to thereby press the whole holder 114 that is mounted so as to contain the cell masses 2, from the lateral direction.

Also, the mounting surface 103a of the above-described culture container 103 is formed to be non-adhesive to cells, and accordingly, the cell mass sheet 4 can be moved by being pressed from the lateral direction.

In addition, it is also acceptable to provide the connection port 103c in the culture container 103, and to circulate the culture liquid in the culture container 103, similarly to the culture liquid supply means 11 that has been described in the first embodiment.

A method for producing the cell mass sheet 4 with the use of the apparatus 1 of the second embodiment will be described below, which has the above-described structure.

Firstly, two steps are performed one of which is a carrying-in step of carrying the accommodating container 5 and/or the culture container 103 in the above-described isolator, and the other one of which is a preparation step of storing the culture liquid in the culture container 103. When the above-described culture container 103 is mounted on the culture container supporting section 7, the moving means 113 of the culture container supporting section 7 are connected to the connecting bars 111a and 112a of the above-described first and second pressing members 111 and 112.

Next, as is shown in FIG. 14(A), a guide placing step is performed that places the above-described guide means G on the above-described culture container 103, and an accommodating step is also performed that accommodates the cell masses 2 in the accommodating portions Ga of the guide means G which has been placed on the culture container 103, by the above-described suction nozzles 8.

After the cell masses 2 have been accommodated in the accommodating portions Ga of the guide means G by the above-described accommodating step, as is shown in FIG. 14(B), a guide retracting step is performed that retracts the guide means G from the above-described culture container 103, and a floating preventing step is further performed that arranges the pressing lid 19 on the upper part of the cell masses 2 which have been arrayed on the culture container 3.

Incidentally, the above-described guide placing step, the above-described guide removing step and the above-described floating preventing step can be performed by an operator or a robot.

Subsequently to the above-described guide retracting step and the above-described floating preventing step, in the above-described culture container supporting section 7, a pressing step is performed that presses the cell masses 2 on the culture container 103 from the lateral directions and brings these cell masses 2 into contact with each other.

That is to say, in a state in which the guide means G has been retracted in the above-described guide retracting step, the cell masses 2 may be arrayed but a clearance may be formed between the cell masses 2; and even though the cell masses 2 are cultured in this state, the fusion of the cell masses 2 may not occur.

Then, firstly, the cell masses 2 are moved in the Y-direction by the first pressing member 111, as is shown in FIG. 14(C), then are moved in the X-direction in the cell masses 2 by the second pressing member 112, and are arrayed in a state in which the cell masses 2 on the culture tray 103 come in contact with each other, as is shown in FIG. 14(D).

When the cell masses 2 have come in contact with each other by the above-described pressing step, the pressing lid 19 is removed which has been provided on the upper part of the above-described arrayed cell masses 2, and the second pressing member 112 is positioned at the retreated position; and then a holder mounting step is performed that mounts the holder 114 on the outer periphery of the above-described arrayed cell masses 2.

When the above-described holder 114 is mounted, the cell masses 2 result in being hindered from moving in the inside of the holder 114, and thereby the cell masses 2 can keep the arrayed state of coming in contact with each other.

Subsequently, the above-described culture container 103 is transferred to the above-described incubator 62, and a culture step is performed in the incubator 62.

When the culture step has been performed for a predetermined period, the cell masses 2 are fused to each other inside of the holder 114, and the cell mass sheet 4 is formed; and then a cell mass sheet moving step is performed that moves the cell mass sheet 4.

In the above-described culture container 103, the cell mass sheet 4 around which the holder 114 is mounted is positioned, and the moving means 115 which is provided in the above-described incubator 62 moves the above-described first and second pressing members 111 and 112, and presses the whole holder 114 that contains the cell mass sheet 4 from lateral directions.

When the cell mass sheet 4 is moved by the cell mass sheet moving step, the culture liquid thereby enters the space between the cell mass sheet 4 and the culture container 103, and accordingly, it becomes possible to supply the culture liquid to the cells which are positioned in the back face side of the cell mass sheet 4.

Incidentally, in the present embodiment, the cell mass sheet 4 in the holder 114 can be moved in the X-direction and the Y-direction by the first and second pressing members 111 and 112, but it is acceptable to connect the moving means 115 to only one of the first pressing member 111 and the second pressing member 112, and to move the cell mass sheet 4 only in one direction of the X-direction and the Y-direction.

FIG. 18 is a view for describing the culture container 103 which can be used in the apparatus 1 for producing the cell mass sheet in the above-described second embodiment, and for describing a variation of the culture container 103 in the above-described second embodiment.

The above-described first pressing member 111 is provided so that both ends thereof can extend and contract due to a pressing force of a spring or the like, and in a state in which the first pressing member 111 is extended, the first pressing member 111 has a width of the cell masses 2 which have clearances formed between each other in a state in which the guide means G has been retracted by the above-described guide retracting step.

Accordingly, when the first pressing member 111 is moved to the advanced position, the first pressing member 111 thereby presses the above-described cell masses 2 in the Y-direction, and can cancel clearances between the arrayed cell masses 2 in the Y-direction.

The above-described first pressing member 111 is structured to be capable of extending and contracting, and accordingly, the second pressing member 112 can be positioned in the closer state, in the state in which the first pressing member 111 is positioned at the advanced position.

Then, the second pressing member 112 presses also the first pressing member 111 while pressing the cell masses 2, and thereby enables the above-described first pressing member 111 to be shortened.

Thereby, the first pressing member 111 capable of extending and contracting can array the cell masses 2 in a state in which both of the first and second pressing members 111 and 112 are positioned at the advanced position, and when the second pressing member 112 is positioned at the advanced position, can prevent the cell masses 2 from resulting in deviating and moving in the Y-direction.

Incidentally, the contents of the above-described first and second embodiments can be appropriately combined with each other.

For instance, in place of the culture tray 3 in the first embodiment, the culture container 103 in the second embodiment is accommodated in the culture container 3; and the through holes are bored in the bottom face of the above-described culture container 103, and also make the pins 14 penetrate thereinto that have been provided on the bottom face of the culture container 3. Then, the pins 14 can be used as the guide means G.

In this case, because the pressing means 105 is provided beside the above-described culture container 103, the cell masses 2 which are accommodated in the accommodating portions Ga of the above-described guide means G do not need to be brought into contact with each other, and accordingly, the cell masses 2 can be easily accommodated in the accommodating portions Ga which have been formed by the pins 14 and the pins 14; and after that, if the above-described pressing means 105 is operated to perform the pressing step, the cell masses 2 can be arrayed in a state of coming in contact with each other.

In addition, by having such a structure, the culture liquid supply means supplies the culture liquid to the back face side of the cell mass sheet 4 by using the through holes which have been provided in the above-described culture container 103 as the supply holes; and thereby the production apparatus can adequately culture the cells that are positioned on the back face side, and can push up the cell mass sheet from the culture container 103 by the above-described pins.

In other words, in the cell mass sheet moving step, the production apparatus does not need to move the cell mass sheet to the lateral directions as in the second embodiment, and accordingly, in the above-described pressing step, can accommodate the culture container 103 in the incubator in a state in which the first and second pressing members 111 and 112 are positioned at the advanced positions; and in this case, it can be omitted to mount the holder 114.

FIG. 20 is a view for describing a culture container 3 which is used in a method for producing a cell mass sheet according to a third embodiment, and can be used, for instance, in the apparatus 1 for producing the cell mass sheet, which has been used in the first embodiment.

The culture container 3 in the present embodiment has a closed-end box shape. In addition, the bottom face thereof constitutes a mounting surface 13a of the cell masses 2, and the mounting surface 13a has non-adhesiveness to cells, similarly to that in the above-described embodiment.

The guide means G in the present embodiment includes a supporting frame 201 that is provided in a lattice form, and pins 202 that are provided so as to hang from the crossing portions in the lattice; and the positions of the crossing portions of the above-described lattice and the positions of the above-described pins 202 are provided in the same arrangement as the pins 14 shown in FIG. 4 in the first embodiment, and four pins 202 are arranged so as to constitute the accommodating portion Ga which accommodates the cell mass 2 therein.

Because the pins 202 are arranged in this way, when the above-described accommodating portions Ga have accommodated the cell masses 2 therein, it becomes possible for the pins to array the cell masses 2 in a state in which the adjacent cell masses 2 come in contact with each other.

Here, the size of each of the lattices that constitute the above-described supporting frame 201 is set so as to be slightly smaller than the diameter of the cell mass 2, because the above-described pins 202 are provided in the above-described array; but the accommodating portions Ga are formed so that the cell masses 2 can be pushed thereinto from the upper part by the above-described suction nozzle 8.

Similarly to the above-described embodiments, when the cell masses 2 have been accommodated in the accommodating portions Ga of the above-described guide means G, the above-described guide means G may be taken out immediately as the guide retracting step, or may also be retracted after the culture of the cell masses 2 has progressed to some extent.

In addition, the cell container 3 in the present embodiment is structured so that a lid 203 is provided on the upper part thereof, but similarly to the above-described embodiments, a pipe 31 of not-shown culture liquid supply means 11 is provided in this lid 203, and is structured so as to supply the culture liquid therethrough.

In addition, in each of the above-described embodiments, the cell masses 2 are planarly arranged on the above-described culture tray and one layer of the cell mass sheet 4 is cultured, but if the plurality of cell masses 2 are layered on the culture tray 3 in the above-described accommodating step, it becomes possible to culture a thicker cell mass sheet 4.

Furthermore, in each of the above-described embodiments, one cell mass 2 is accommodated in each of the accommodating portions Ga of the above-described guide means G, but a plurality of cell masses 2, for instance, approximately two to four pieces of cell masses 2 of a previously set number may also be accommodated in one accommodating portion Ga. Each of the accommodating portions Ga may individually accommodate a predetermined number of cell masses 2 therein.

### [Reference Signs List]

- 1: Apparatus for producing cell mass sheet
- 2: Cell mass
- 3: Culture container
- 4: Cell mass sheet
- 5: Accommodating container
- 8: Suction nozzle
- 9: Nozzle moving means
- 10: Inspection means
- 11: Culture liquid supply means
- 12: Cell mass sheet moving means
- 13: Culture tray
- 13a: Mounting surface
- 13b: Through hole
- 13c: Supply hole
- 14: Pin
- 19: Pressing lid
- G: Guide means
- Ga: Accommodating portion

## Claims

1. An apparatus for producing a cell mass sheet, which produces a cell mass sheet in which a plurality of cell masses that have been planarly arranged on a mounting surface in a culture container are fused to each other by being cultured, **characterized in that**:
guide means that has a plurality of accommodating portions which can individually accommodate the cell masses therein, and supply means that supplies the cell masses into the accommodating portions of the guide means which has been placed on the mounting surface;
wherein the supply means arrays the cell masses on the mounting surface, and then the guide means is retracted from above the mounting surface.

2. The apparatus for producing the cell mass sheet according to claim 1, **characterized in that**
the supply means comprises:
holding means that holds cell masses thereon and moving means that moves the holding means; and
when the moving means has moved the holding means and taken out the cell masses from the accommodating container that accommodates the cell masses therein, the cell masses are accommodated in the accommodating portions of the guide means on the mounting surface.

3. The apparatus for producing the cell mass sheet according to claim 1 or claim 2, **characterized in that** the guide means is placed on the mounting surface, and guide moving means is provided that retracts the guide means from above the mounting surface.

4. The apparatus for producing the cell mass sheet according to any one of claim 1 to claim 3, **characterized by** further comprising pressing means that presses the cell masses which are arrayed on the mounting surface from a lateral direction, after the guide means has been retracted from above the mounting surface.

5. The apparatus for producing the cell mass sheet according to any one of claim 1 to claim 4, **characterized in that** the guide means is formed of a plurality of pins that are provided so as to be positioned in the periphery of each of the cell masses which are arrayed on the mounting surface, and the accommodating portions are formed by spaces between the plurality of pins.

6. The apparatus for producing the cell mass sheet according to claim 5, **characterized in that**:
a culture tray that mounts the cell masses thereon is provided in the culture container, and the mounting surface is formed on the culture tray;
a plurality of through holes that correspond to the arrangement of the plurality of pins are bored in the culture tray;
the guide means is placed on the mounting surface by making the plurality of pins project from the through holes; and
the guide means is retracted from above the mounting surface by making the plurality of pins sink in the through holes.

7. The apparatus for producing the cell mass sheet according to claim 6, **characterized in that**
the plurality of pins are installed vertically on the bottom face of the culture container, and
the apparatus for producing the cell mass sheet further comprises guide moving means that moves the culture tray downward relatively to the culture container and thereby places the plurality of pins on the mounting surface, and moves the culture tray upward relatively to the culture container and thereby retracts the plurality of pins from the mounting surface.

8. The apparatus for producing the cell mass sheet according to any one of claim 1 to claim 7, **characterized by** further comprising floating preventing means that prevents the cell masses that are arrayed on the mounting surface from floating up.

9. The apparatus for producing the cell mass sheet according to any one of claim 1 to claim 8, **characterized in that** the mounting surface has properties of being non-adhesive to cells.

10. A method for producing a cell mass sheet, which produces a cell mass sheet in which a plurality of cell masses that have been planarly arranged on a mounting surface in a culture container are fused to each other by being cultured, **characterized by** comprising:
a guide placing step of mounting guide means that has accommodating portions regularly formed therein which can individually accommodate the cell masses therein, on the mounting surface;
an accommodating step of accommodating the cell masses in the accommodating portions of the guide means, respectively, and arraying the cell masses on the mounting surface;
a guide retracting step of making the guide means retract in a state in which the cell masses are arrayed on the mounting surface; and
a culture step of culturing the cell masses that are arrayed on the mounting surface, and making the cell masses fused to each other.

11. The method for producing the cell mass sheet according to claim 10, **characterized in that**
the guide placing step provides a plurality of pins that act as the guide means, so as to be positioned in the periphery of each of the cell masses which are arrayed on the mounting surface, and spaces among the plurality of pins constitute the accommodating portions; and
in the accommodating step, the spaces among the plurality of pins accommodate the cell masses therein.

12. The method for producing the cell mass sheet according to claim 11, **characterized in that** in the accommodating step, when the cell masses are accommodated in the spaces among the plurality of pins, which constitute the respective accommodating portions, the cell masses that are accommodated in the adjacent accommodating portions come in contact with each other.

13. The method for producing the cell mass sheet according to claim 12, **characterized by** further comprising culturing the cell masses which are in a state of being accommodated in the accommodating portions of the guide means, prior to the guide retracting step.

14. The method for producing the cell mass sheet according to claim 10 or claim 11, **characterized by** further comprising a pressing step of pressing the arrayed cell masses from lateral directions to bring the adjacent cell masses into contact with each other, in between the guide retracting step and the culture step.

15. The method for producing the cell mass sheet according to any one of claim 10 to claim 14, **characterized in that** the mounting surface has non-adhesive properties for cells.
